(19) Europäisches Patentamt
European Patent Office
Office européen des brevets

(11) Publication number : **0 479 640 A2**

(12) # EUROPEAN PATENT APPLICATION

(21) Application number : **91402517.6**

(22) Date of filing : **23.09.91**

(51) Int. Cl.$^5$ : **C07H 19/10,** C07H 19/20, A61K 31/70, C07H 19/04

(30) Priority : **28.09.90 EP 90402695**

(43) Date of publication of application : **08.04.92 Bulletin 92/15**

(84) Designated Contracting States : **AT BE CH DE DK ES FR GB GR IT LI LU NL SE**

(71) Applicant : **MERRELL DOW PHARMACEUTICALS INC.**
**P.O. Box 156300 2110 East Galbraith Road**
**Cincinnati Ohio 45215-6300 (US)**

(72) Inventor : **Halazy, Serge**
**2 Rue Hans Haug, Wolfisheim**
**F-67200 Strasbourg (FR)**
Inventor : **Casara, Patrick**
**12 Rue de la Scierie**
**F-67117 Ittenheim (FR)**
Inventor : **Neises, Bernhard**
**Flösserweg 5**
**W-7600 Offenburg-Griesheim (DE)**
Inventor : **Jund, Karin**
**7 Boulvard Clemenceau**
**F-67000 Strasbourg (FR)**

(74) Representative : **Gillard, Marie-Louise et al**
**Cabinet Beau de Loménie 55, Rue d'Amsterdam**
**F-75008 Paris (FR)**

(54) **Novel phosphonate derivatives of certain nucleosides.**

(57) This invention relates to novel phosphonate derivatives of certain nucleoside analogs, to the methods for their preparation and to their use as anti-viral agents.

EP 0 479 640 A2

This invention relates to novel phosphonate derivatives of certain nucleoside analogs, to the methods for their preparation and to their use as anti-viral agents.

More specifically this invention relates to compounds of the formula

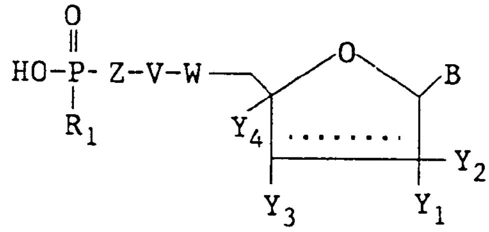

I

their tautomeric forms, their optical and geometric isomers, and mixtures thereof, and the pharmaceutically acceptable salts thereof,

wherein the dotted line represents a facultative double bond,

$R_1$ is $C_{1-10}$ alkyl, $CH_{3-x}F_x$ , $C_{1-10}$ alkyl-OH or $OR_2$, x is zero, 1, 2 or 3,

$R_2$ is H or $C_{1-6}$ alkyl,

Z is O, $CH_2O$, $CF_2$, $CCl_2$, CHF, CHCl, CFCl or is deleted,

V is $(CH_2)_m$ or $(CH_2)_m$ Y-$(CH_2)_n$, or is deleted, with m and n being zero, 1, 2, 3 or 4, and Y is -C≡C,

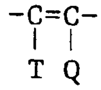 , 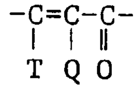 , 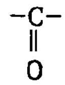 , 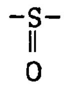 , 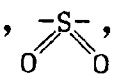 , 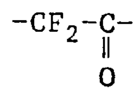 , 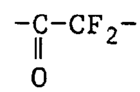 ,

or

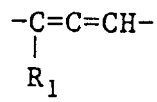 ,

T and Q each are H, F or Cl,

W is O or is deleted,

$Y_1$ is H, Cl, F or OH,

$Y_2$ is H, Cl or F, with the proviso that when the dotted line represents a double bond, then $Y_3$ is F, Cl, or H, $Y_1$ is H, F or Cl and $Y_2$ is deleted,

$Y_3$ is $N_3$, F, Cl, OH or H,

$Y_4$ is H or $N_3$, with the proviso that when $Y_3$ is $N_3$, $Y_4$ is H, with the provisos that

(a) at least one of Z, V and W moieties is other than deleted,

(b) the -Z-V-W- moiety is other than oxygen,

(c) the -Z-V-W- moiety does not contain an oxygen to oxygen bond,

(d) when $Y_3$ is $N_3$, and the -Z-V-W- moiety is $CH_2$ or $-CH_2O-$, then $R_1$ is other than $OR_2$,

(e) when B is a pyrimidine nucleic base and $Y_4$ is H, then the -Z-V-W- moiety is other than $CH_2O$, and

(f) when the -Z-V-W- moiety is a $C_{1-5}$ alkylene and both $Y_3$ and $Y_4$ are other than $N_3$, then $R_1$ is other than $OR_2$,

B is a base selected from the group consisting of

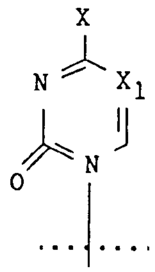

(a)

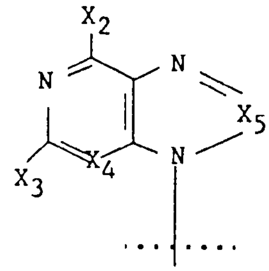

(b)

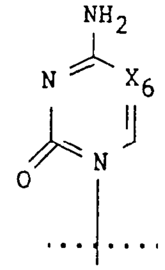

(c)

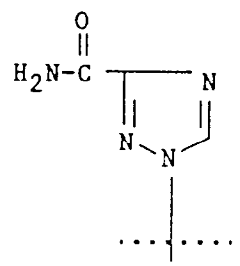

(d)

and

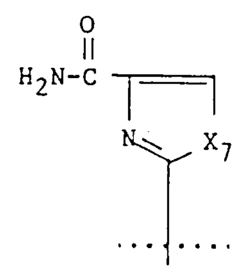

(e)

X is $OR_2$,
$X_1$ is N or C-$Y_7$ with $Y_7$ being H, $CH_3$, $C_2H_5$, I, F, Cl, Br, NHR, SR, -C≡CH, $CH=CH_2$, CH=CHBr, $CH_2CH_2Cl$, $-CH_2CH_2F$, or $CH_2C_6H_4OCH_2C_6H_5$ (meta), and R is H, $CH_3$, $C_2H_5$, $CH_2C≡CH$, $-CH_2CH=CH$ or $CH_2CN$,
$X_2$ is H, OH, Cl, $NH_2$, $SCH_3$ or SH,
$X_3$ is H, $NH_2$, F, Cl, Br or I,
$X_4$ is N or CH,
$X_5$ is N or C-$Y_5$ with $Y_5$ being H, OH, $NH_2$, $NHNH_2$, $CH_3$, Cl, Br, or NHMe,
$X_5$ is C-$Y_6$ with $Y_6$ being H, F, $CH_3$ or CH=CHBr,
$X_7$ is S or Se.

The term "pharmaceutically acceptable salts" embraces both acid addition salts and metal and amine salts which are known to be non-toxic and useful derivatives in the preparation of pharmaceutical formulations suitable for end-use applications.

Pharmaceutically acceptable acid addition salts include the known non-toxic organic or inorganic acid addition salts of the base compounds of Formula I. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic forms, demonstrate higher melting points and an increased stability.

Pharmaceutically acceptable metal and amine salts are those salts which are stable under ambient conditions, and wherein the cation does not contribute significantly to the biological activity of the salt. Suitable metal salts include the sodium, potassium, calcium, barium, zinc, and aluminium salts. The sodium and potassium salts are preferred. Suitable amine salts are prepared from amines which have sufficient basicity to form a stable salt, and preferably include those amines which are frequently used in medicinal chemistry because of their low toxicity and acceptability for medical use. These include the trialkylamines such as triethylamine, and others including procaine, dibenzylamine. N-benzyl-betaphenethylamine, ephenamine, and N,N′-dibenzylethylenediamine, dehydroabietylamine, N-ethylpiperidine, benzylamine, and dicyclohexylamine.

The terms $C_{1-6}$ alkyl or $C_{1-10}$ alkyl include the straight and branched chain hydrocarbonyls having the designated number of carbon atoms, including such radicals as methyl, ethyl, propyl, isopropyl, butyl, t-butyl, pentyl, hexyl and the like. When used, the term halogeno embraces F, Cl, Br and I.

The isomeric forms of the compounds of Formula I include the optical and geometric forms which are well-known entities and may be prepared by known processes designed to enrich the production of the desired geometrical isomers and/or optical isomers. However, mixtures may be resolved or isolated according to conventional and standard procedures well known in the art, e.g., chromatographic separation, fractional crystallization, use of optically active acids, enzymatic resolution and the like. Tautomeric enol-keto forms may exist at the 6-position of the purine nucleus, and the pyrimidine will exhibit amine-imine tautomeric forms.

The compounds of Formula I are depicted in a non-specific representation with respect to stereochemical configuration but it is to be understood that the cisstereochemical form relating to the base and the substituent located at the 4-position of the ribose moiety is preferred.

It is to be noted that the moieties such as

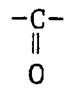 , 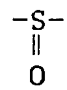 ,

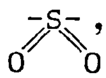 , 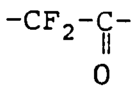 , 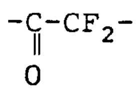 , 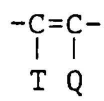 , 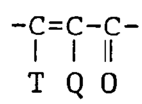 , and 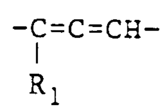 ,

may also be written as C(O), S(O), $S(O)_2$, $CF_2C(O)$, $C(O)CF_2$, C(T)=C(Q), and C(T)=C(Q)C(O) and -$C(R_1)$=C=CH, respectively, and the

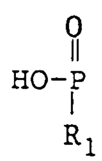

moiety may be written as

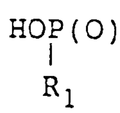

or as $(HO)(R_1)P(O)$.

Quite obviously from the fact that Y may be

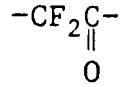 ,

or

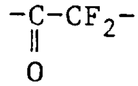 ,

it is important that the defined moieties of -Z-V-W- be read as shown. For example, if Z and W are deleted, and V is $(CH_2)_mY\text{-}(CH_2)_n$, with m and n each being 1 and Y is $-CF_2C(O)-$, the

$$\underset{\displaystyle R_1}{\overset{}{HO\underset{|}{P}(O)}}-Z-V-W-$$

moiety would be

$$\underset{\displaystyle R_1}{HO\underset{|}{P}(O)}CH_2CF_2C(O)CH_2-.\ \text{It would not be}\ \underset{\displaystyle R_1}{HO\underset{|}{P}(O)}CH_2C(O)CF_2CH_2-.$$

Similarly, if Z and W were deleted, and V was stated to be $CH_2C(T)=C(Q)C(O)CH_2$, the correct alignment would be $(HO)(R_1)P(O)CH_2C(T)=C(Q)C(O)CH_2-$ whereas $(HO)(R_1)P(O)CH_2C(O)C(Q)=C(T)-CH_2$ would be an incorrect interpretation.

Representative of the most preferred moieties of the depicted bases of formulae (a), (b), (c), (d) and (e) for defining compounds of Formula I are:
guanine,
adenine,
pyrrolo[2,3-d]pyrimidine,
pyrazolo[3,4-d]pyrimidine,
8-azapurine,
benzoguanine,
2-aminopurine,
8-hydroxy-purine, adenine or guanine,
8-methyl-purine, adenine or guanine,
8-chloro-purine, adenine or guanine,
8-bromo-purine, adenine or guanine,
8-iodo-purine, adenine or guanine,
8-amino-purine, adenine or guanine,
8-methylamino-purine, adenine or guanine,
8-dimethylamino-purine, adenine or guanine,
5-fluorouracil,
5-methyluracil,
5-hydroxyuracil,
5-bromouracil,
5-iodouracil,
5-chlorouracil,
5-nitrouracil,
5-(2-bromovinyl)uracil,
5-hydrroxymethyluracil,
5-fluoro-cytosine,
5-bromo-cytosine,
5-iodo-cytosine,
5-nitro-cytosine,
2-amino-6-chloropurine,
5-fluoro-4-(methylthio)pyrimidin-2(IH)one,
5-bromo-4-(methylthio)pyrimidin-2(IH)one,
5-iodo-4-(methylthio)pyrimidin-2(IH)one,
5-aza-cytosine,
2,6-diaminopurine,
xanthine,
hypoxanthine

In practice, it is preferred to employ bases having, e.g., either a pyrimidine or a purine nucleic base (including their tautomeric forms). Preferred pyrimidine type bases are uracil, thymine, cytidine, 5-halogenated (F, Br

or I) or 5-carboxy uracils, and 5-methyl or 5-hydroxymethylcytidines. Preferred purine type bases are adenine, guanine, 6-methylamino or 6,6-dimethylamino purines or 6-OH purines, 8-azaguanine, and 6-oxy or 2,6-dioxy purines. Otherwise stated, preferred compounds of this invention are those which contain such nucleosidic moieties as adenosine, deoxyadenosine, guanosine, deoxyguanosine, cytidine, deoxycytidine, uridine, deoxyuridine, thymidine, inosine and xanthosine.

In general, the compounds of this invention may be prepared by the application of analogous chemical reactions known in the art using reactants which are already known or which may be prepared using standard processes and techniques well known in the art. A handy reference work may be found in Comprehensive Organic Chemistry, Vol.5 (Biological Compounds) as edited by E. Haslam (Pergamon Press, 1979) and the large body of literature which followed.

Typically the preparation of the desired compounds of Formula I may be effected by a condensation of an activated nucleophile with an electrophile bearing a suitable leaving group. Of course, in those instances wherein there are reactive moieties present on the reactants which would interfere with the efficacy of the desired reaction, then such reactive groups may be suitably protected by techniques and processes well known in the art. This is particularly true in protecting the hydroxyl groups of the ribose moiety or the carbonyl groups of the "Y" substituents. Additionally, it is preferred to utilize those reactants wherein the nucleic base bears substituents which will survive the reactions necessary for effecting the condensation reactions and then, following completion of the condensation, the nucleic bases may be modified (either before or after the de-esterification of the phosphonate esters) to produce the desired compounds. Further, it is also obvious that, depending on the economics of any particular set of reactants it may be convenient to effect separation of any isomeric forms resulting from any particular step; the separation being effected by standard techniques (e.g. HPLC) well known in the art.

In essence the particular set of reactants to be employed for the preparation of a given compound will depend - to a large degree - upon whether the condensation reaction will produce a carbon-to-oxygen bond or whether it will produce a carbon-to-carbon bond. In the first instance the activated nucleophile will be on the reactant bearing the ribonucleoside moiety whilst the electrophile bearing the suitable leaving group will be borne by the reactant having the protected phosphonate moiety. In the situation wherein a carbo-n-to-carbon bond is produced the converse would generally be true.

The reaction resulting in a carbon-to-oxygen bond may be illustrated in the following depicted reaction scheme

REACTION SCHEME A

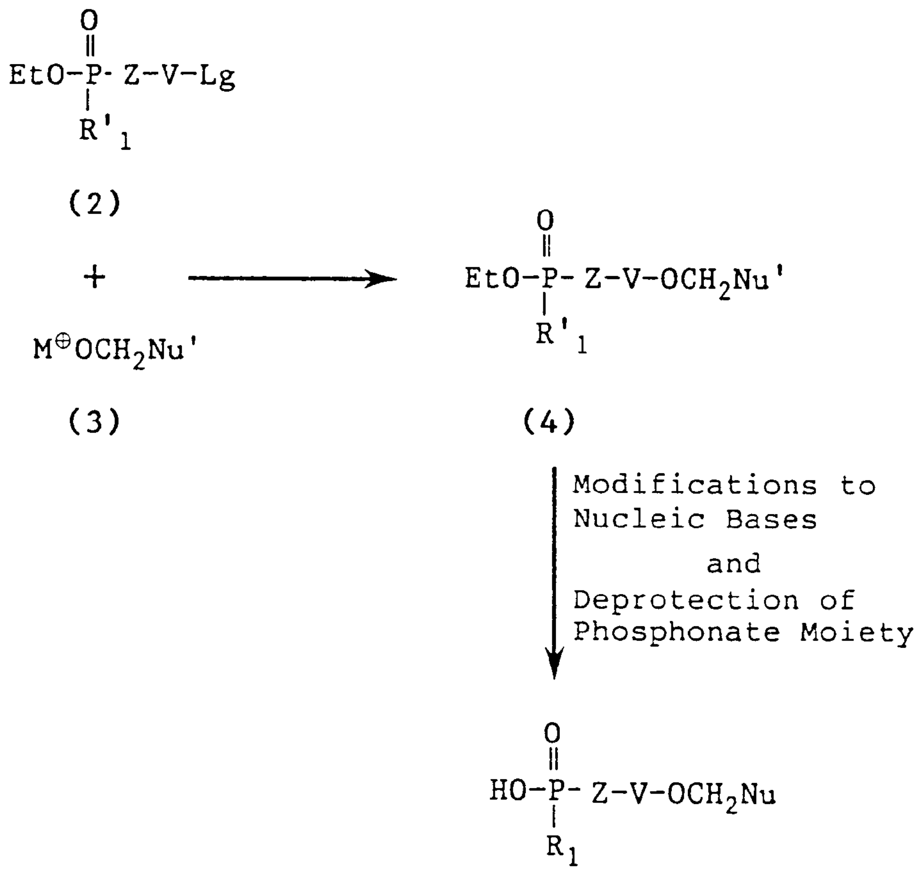


(2)

+

(3)

(4)

(5)

wherein Et is ethyl, Lg represents a suitable leaving group, M⊕ is a metallo cation, $R_1$, Z, V are as previously defined, $CH_2Nu$ represents the nucleoside of Formula I having the formula

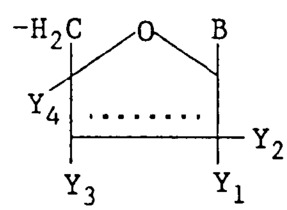

(6)

wherein the dotted line, B, $Y_1$, $Y_2$, $Y_3$, and $Y_4$ are as defined for Formula I. Similarly, the $H_2CNu'$ embraces compounds of the formula

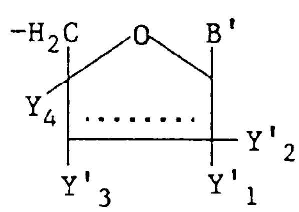

(7)

which are the reaction-protected and modified analogs of Formula (6) wherein the primed moieties (e.g., B′, $Y'_1$, $Y'_2$, $Y'_3$) are as defined for their un-primed moieties of Formula (6) and/or modified analogs which are transformable to the desired moieties [as defined in Formula (6)] once the necessary condensation reactions have taken place. $R'_1$ is as defined for $R_1$ or is a reaction-protected analog thereof which, following the condensation, is transformable to the desired $R_1$ moiety.

Suitable leaving groups for the foregoing reaction include such groups as triflates, alkyl or aryl sulfonates (e.g. $CF_3SO_2O$, tosylate and mesylate) and halides (preferably bromo, chloro or iodo). Suitable metallo cations are Na, K, Li, Cs, Mg, ZnBr or MgBr, preferably when cesium is utilized the condensation is effected in the presence of a crown ether. In effecting the foregoing reactions, the reactants (2) and (3) are contacted together in a suitable anhydrous solvent (e.g. tetrahydrofuran (THF), dimethylformamide (DMF), ether and the like at temperatures generally known for such type reactions, the range being -78°C (e.g. when a lithio derivative is reacted with a bromide in THF) to about room temperature, the reaction generally being conducted in an inert atmosphere using nitrogen or argon. Once the condensation is effected, modifications to the nucleosidic moiety are completed, if necessary, and then the protecting groups, if any, are removed to obtain the desired compounds of Ia. If desired, the deprotection of the phosphonate esters may be effected prior to the modifications, if necessary, of the nucleic bases.

The preparation of the ribonucleosides of Formula (6) and their reaction-protected or modified versions of Formula (7) are well known compounds which are prepared by processes and techniques well known and well understood by those in the art; such compounds, processes and techniques being disclosed in such many references as the reference work edited by Evans. In general the route synthesis for any individual compound will depend on the specific substituents of each molecule and upon the ready availability of intermediates necessary; again such factors being appreciated by those of ordinary skill in the art.

In the special situation wherein the compounds of formula I contain a 4′-azido moiety, the preparation of the 4′-azido ribonucleosides may be effected by the method published by Maag, et al. in Antiviral Research Supplement 1 (1981) entitled "4′-Azicothymidine: Synthesis and *in vitro* anti-HIV activity". Essentially the process for preparing the necessary intermediates may be illustrated by the generic scheme

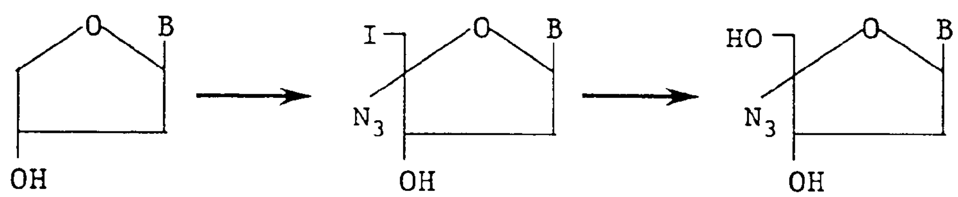

The synthesis of the 4′-azido ribonucleoside is effected by the addition of iodine azide to the exocyclic double bond of 2′,5′-dideoxynucleoside-4′-enes to provide the 4′-azido-2′,5′-dideoxy-5′-iodo derivatives. Acylation of the 3′-OH group followed by an oxidative iodine displacement reaction (using a peracid salt) and a deprotection will yield the corresponding 4′-azido-2′-deoxynucleosides.

Exemplary of the type of transformation of the precursors (3) is the situation wherein it is desired to produce a compound in which at least one $Y_1$ and $Y_3$ is OH, the 5′OH moiety would first be selectively protected by the treatment with one equivalent of t-butyldimethylsilyl chloride, and then any OH may be protected by such protecting groups as, for example, acetyl, benzyl, benzoyl or tetrahydropyran. The 5′O-t-butyldimethylsilyl moiety is selectively removed with tetrabutyl ammonium fluoride ($Bu_3NF$) in THF to yield the 5′OH function which is then activated according to standard procedures to yield the appropriate reactant for the condensation reaction of Reaction Scheme 1.

The art teaches that there are numerous starting materials which may be utilized in the preparation of the $X_{1-7}$-substituted versions of the purine, pyrimidine, triazole and imidazole nucleic bases of sub-formulae (a) to (e) of Formula I, and therefore the $X'_{1-7}$ precursor moieties (of Formula (3), (4) and (7) are those which are as defined for the $X_{1-7}$ moieties of Formula I and will remain intact during the necessary processes to efficiently effect the condensation reations or are modified versions which are transformable to the desired moiety once the condensation reactions have taken place. For example, if a chloro substituent is to be in the final compound, then the $CH_2Nu'$ reactant would include a chloro moiety for that could be left intact throughout the required series of reactions. On the other hand, for example, one could use a 6-chloro or a 2,6-dichloro purine starting compound and following the condensation reactions they could be transformed into a 6-amino or a 2,6-diamino final product, respectively, by reaction with the appropriate amines (e.g., $NH_3$, -HN(H)(R) or -HN(R)(R) in methanol under pressure at about 100°C. Similarly, in the instance wherein the X substituent is OR, the corresponding

6-chloro purine moiety may be reacted with an acid (HCl/HOH) to obtain the corresponding 6-OH analog or with an alcohol in basic conditions to obtain the corresponding 6-OR analog. In the same vein, X′ may be a protected OH group using such groups as $-OCH_3$, $OCH_2CH_2OCH_3$, Obenzyl, O-C(O)phenyl, or -O-C(O)methyl and then such moieties would be transformed to the desired 6-OH moieties. Analogously, a compound wherein X is to be an amine, the precursor transformable thereto could be an amide (e.g., NHC(O)phenyl), an amidine (e.g.

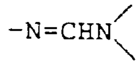

or $-N(C(O)phenyl)_2$, said moieties being transformable to the desired amine by standard procedures. Similarly, a 6-chloro purine starting material may be transformed to the desired $-SCH_3$ moiety by treatment with methyl mercaptan using standard procedures. These reactions, and the proper sequencing of such reactions, all follow principles well understood and practised by those skilled in the art and all steps used processes well known in the art, as well as other factors such as the ready availability of the appropriate starting materials, reactants and the volume capacity of compound to be produced are also factors which are appreciated by those skilled in the art for choosing any particular reaction scheme.

Exemplary of the Z-V-W moieties to which the foregoing Reaction Scheme A would be applicable are those wherein

(a) Z is $CH_2O$, V is as generally defined and W is O,

(b) Z is $CH_2O$ and V and W are deleted,

(c) Z is $CF_2$, $CCl_2$, CHF or CHCl, V is as generically defined and W is O, and

(d) Z is deleted, V is as generically defined and W is O.

(In the instances of (b) and (d) it is understood that when Z-V-W is $CH_2O$, then such moiety (by provisos

(d) and

(e) of Formula I) may be outside of the scope of this invention.)

Of course in those instances wherein V contains a carbonyl moiety which may interfere with the reaction, e.g. V is

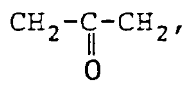

the carbonyl is reduced to its alcohol, the alcohol protected with a trimethylsilyl ether, and following the condensation the silyl ether is cleaved to its alcohol and the alcohol is oxidized (preferably via a Swern oxidation) to the desired ketone.

A variation of the foregoing process is the process depicted by the following generic reaction scheme

REACTION SCHEME B

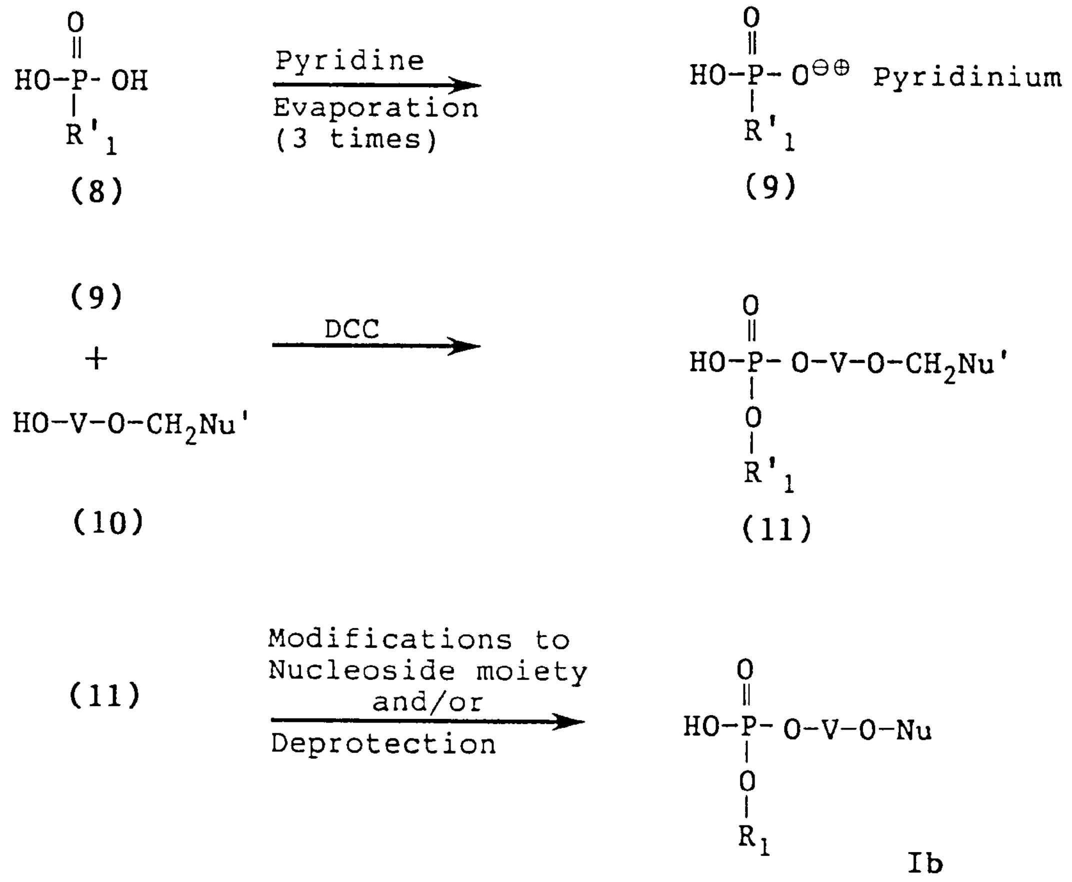


wherein V, $R_1$, the $-CH_2Nu'$ and $-CH_2Nu$ moieties being as previously defined, and $R'_1$ is as defined for $R_1$ or is a reaction-protected analog thereof which, following the reactions necessary to complete the condensation, is transformable to the desired $R_1$ moiety.

The foregoing reaction is particularly well suited to those compounds of Formula I wherein Z and W are both oxygen and V is as generically defined in Formula I. In effecting this condensation the phosphonate salt (9) is coupled with the appropriate nucleoside (10) by interaction at about 20°C to 40°C for one to three days in the presence of 3 to 5 equivalents of decyclohexylcarbodiimide (DCC) under anhydrous conditions, preferably in an inert atmosphere (nitrogen or argon being preferred). After the reaction is completed it is quenched with water, the N,N-dicyclohexylurea is filtered, the filtrate washed with diethyl ether and the products are isolated and purified using recrystallization or high performance liquid chromatography (HPLC) techniques. With HPLC, it is preferred to use a reverse phase on a strong anionic column. Following the condensation the modifications to the nucleic bases and deprotection of the phosphonate moiety may be effected.

In those instances wherein the nucleophilic alkylation process produced a carbon-carbon bond at the site of reaction, the following procedure may be utilized.

REACTION SCHEME C

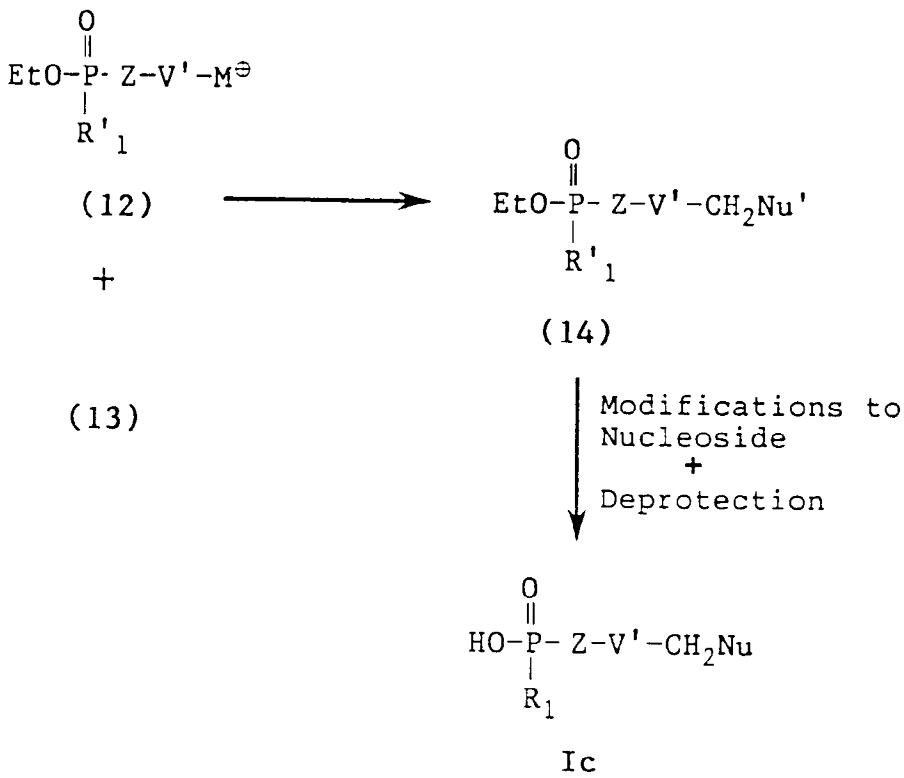


(12) + (13) → (14) → Ic

wherein

V′ is as defined except it cannot be deleted or is a reaction-protected analog thereof, which, following the completion of the condensation reactions, will be transformable to the desired V moiety.

Z, $R'_1$, Et, Lg, Nu and Nu′ are as previously defined.

Exemplary of the Z-V moieties to which the foregoing reaction scheme would be applicable are:

O-V- wherein V is other than deleted;

$-CH_2O$-V- wherein V is other than deleted;

$CF_2V$, $CCl_2V$, CHFV or CHCl-V wherein V is other than deleted;

$CF_2$, $CCl_2$, CHF, CHCl, and V wherein it is other than deleted.

Of course, in certain instances the process of Reaction Scheme C is modified to accomodate the known properties of the reactants. For example, to prepare a compound of Formula I wherein the Z-V-W moiety is CHF it is preferred to prepare a lithio derivative of a tetraethyl fluoromethylene bisphosphonate which is reacted with an aldehyde derivative of the $CH_2Nu'$(i.e.,

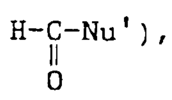

and the resulting olefin of the condensation reaction is chemically reduced and the resulting product deprotected and modified to the desired product. Alternatively an ester derivative of the $CH_2Nu$(i.e.,

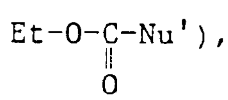

moiety may also be utilized in a condensation reaction with a metallo derivative of the phosphonate (9) and the resulting product is chemically reduced, the product of which is then deprotected and modified to produce the desired compounds of Ic.

Of course, in those instances of Reaction Scheme C wherein it is desired, homologation procedures well

known in the art may be employed to elongate the alkylene moiety of the 5′-nucleoside (i.e., $CH_2Nu$). A suitable process may be illustrated as:

$$HOCH_2Nu \rightarrow CNCH_2Nu \rightarrow HO(CH_2)_2Nu \rightarrow CN(CH_2)_2Nu— HO(CH_2)_3Nu$$

and so on.

In the particular instance wherein $R_1$ is $OR_2$, Z is deleted, m is zero and Y is -CH=CH-, i.e., to produce compounds having an unsaturated carbon atom to which the phosphonate moiety is attached, e.g., a compound of the formula

$$(HO)_2P(O)\text{-}CH{=}CH(CH_2)_nNu \qquad (15)$$

another special reaction sequence may be utilized. For example, a compound of formula $HO(CH_2)_nCH_2Nu'$ may be oxidized by known methods (e.g., Swern oxidation, Moffatt method, PDC or PCC, or with the use of tetrapropylammonium perruthenate with N-methylmorpholine N-oxide to yield the corresponding aldehyde, condensation of the aldehyde with the anion of a bisphosphonate $\{[(OR_2)_2P(O)]_2CH^{\ominus}\}$ obtained by reacting the bis-phosphonate with NaOH or LDA in THF or DMF to yield the desired product (15). This reaction may be alternatively effected in a one-pot reaction using the procedures described by Montgomery (J. Med. Chem. 22, 109 (1979) and by Ireland (J. Org. Chem., 50, 2200 (1985).

The following specificexamples will serve to adequately illustrate the types of processes generically described above but such examples are not intended to be limiting; it being obvious to one of ordinary skill in the art that such may be adapted to suit any given pair of reactants for the preparation of the compounds of this invention.

EXAMPLE 1

5′-O-[3-O-(Trifluoromethylphosphinico)-3-hydroxy-2-oxopropyl]-3′-fluoro-2′,3′-dideoxy-5-chlorouridine

Step A:

To a stirred solution of 3′-fluoro-2′,3′-dideoxy-5′-chlorouridine (1.05 g, 4 mmol) in dimethyl sulfoxide (20 ml), sodium hydride (0.17 g, 8 mmol) is added. The stirring is continued at room temperature under nitrogen and the mixture is treated with α′-tetrahydropyranyloxy-α-bromoacetone (0.96 g, 4 mmol). The stirring is continued for 24 hours at room temperature. The mixture is neutralized with acetic acid and concentrated *in vacuo*. The residue is diluted with ethanol and p-toluenesulfonic acid (0.1 g) is added and stirred overnight at room temperature. The mixture is concentrated and purified by flash chromatography on silica gel with ethyl acetate: ethanol, 95:5 as an eluent to yield 5′-O-(3-hydroxy-2-oxopropyl)-3′-fluoro-2′,3′-dideoxy-5-chlorouridine. Yield: 0.9 g, 75%.

Step B:

5′-O-(3-hydroxy-2-oxopropyl)-3′-fluoro-2′,3′-dideoxy-5-chlorouridine (0.31 g, 1 mmol) and trifluoromethyl phosphonic acid (0.3 g, 2 mmol) are dissolved in anhydrous pyricine (10 ml) and the solvent is removed in partial vacuum at 35°C. This procedure is repeated twice to provide an anhydrous reaction mixture. The residue is dissolved in anhydrous pyridine (20 ml) and dicyclohexylcarbodiimide (0.82 a, 4 mmol) is added. After stirring at 35°C under nitrogen atmosphere for 20 hours, water (10 ml) is added, dicyclohexyl urea is filtered off and the pH of the filtrate is adjusted to 7 with 1M ammonium hydroxyde. The product is purified by ion exchance chromatography (DOWEX AG 1X4), eluted with 4N formic acid to yield 5′-O-[3-O-(trifluoromethylphosphinico)-3-hydroxy-2-oxopropyl]-3′-fluoro-2′,3′-dideoxy-5-chlorouridine. Yield: 0.25 g, 55%.

EXAMPLE 2

5′-O-[4-O-(-n-Butylphosphinico)-4-hydroxy-2-butynyl]-2′,3′-dihydro-3′-deoxythymidine

Step A:

To a stirred solution of 2′,3′-dihydro-3′-deoxythymidine (0.43 g, 2 mmol) in dimethylsulfoxide (10 ml), sodium hydride (0.85 g, 4 mmol) is added, the stirring continued at room temperature under nitrogen and the mixture is treated with 1-bromo-4-tetrahydropyranyloxy-2-butyne (0.47 g, 2 mmol). The stirring is continued for 24 hours at room temperature. The mixture is neutralized with acetic acid and concentrated *in vacuo*. The residue is taken up in ethanol and p-toluene sulfonic acid is added (0.05 g) and stirred overnight at room temperature. The mixture is then concentrated and purified by flash chromatography on silica gel with ethyl acetate:

ethanol, 95:5 as an eluent to yield 5′-O-(4-hydroxy-2-butynyl)-2′,3′-dihydro-3′-deoxythymidine. Yield: 0.15 g, 60%.

Step B:

5′-O-(4-hydroxy-2-butynyl)-2′,3′-dihydro-3′-deoxythymidine (0.13 g, 0.5 mmol) and n-butylphosphonic acid (0.14 g, 1 mmol) are dissolved in anhydrous pyridine (5 ml) and the solvent is removed in partial vacuum at 35°C. The residue is dissolved in anhydrous pyridine (10 ml) and dicyclohexylcarbodiimide (0.4 g, 2 mmol) is added. After stirring at 35°C under nitrogen atmosphere for 20 hours, water (10 ml) is added and dicyclohexylurea is filtered off. Then the pH oF the mixture is adjusted to 7 with 1M ammonium hydroxyde and the product is purified by ion exchange chromatography (DOWEX AG 1 x 4), eluted with 4N formic acid to yield 5′-O-[4-O-(-n-butylphosphinico)-4- hydroxy-2-butynyl]-2′,3′-dihydro-3′-deoxythymidine. Yield: 0.11 g, 50%.

EXAMPLE 3

5′-O-[3-O-(Methylphosphinico)-3-hydroxypropyl]-3′-azido-3′-deoxythymidine

Step A:

To a stirred solution of 3′-azido-3′-deoxythymidine (1.25 g, 5 mmol) in dimethylsulfoxide (30 ml), sodium hydride (0.2 g, 10 mmol) is added, the stirring is continued at room temperature under nitrogen and the mixture is treated with 3′-tetrahydropyranyloxy-1-iodopropane (1.35 g, 5 mol). The stirring is continued for 24 hours at room temperature. The mixture is neutralized with acetic acid and concentrated *in vacuo*. The residue is diluted with ethanol and p-toluenesulfonic acid (0.1 g) is added and stirred overnight at room temperature. The mixture is concentrated and purified by flash chromatography on silica gel with ethylacetate: ethanol, 95:5 as an eluent to yield 5′-O-(3-hydroxypropyl)-3′-azido-3′-deoxythymidine. Yield: 1.25, 65%.

Step B:

A solution of dicyclohexylcarbodiimide (2.5 g, 12 mmol) in anhydrous pyridine (5 ml) is added to a solution of 5′-O-(3-hydroxypropyl)-3′-azido-3′-deoxythymidine (0.92 g, 3 mmol) and methylphosphonic acid (0.58 g, 6 mmol) in anhydrous pyridine (10 ml). After stirring at 35°C under nitrogen atmosphere for 20 hours, water (10 ml) is added, dicyclohexylurea is filtered of: and the pH of the filtrate is adjusted to 7 with 1M ammonium hydroxyde. The product is purified by ion exchange chromatography (DOWEX AG 1 X 4), eluted with 4N formic acid to yield 5′-O-[3-O-(methyl-phosphinico)-3-hydroxypropyl]-3′-azido-3′-deoxythymidine. Yield: 0.65 g, 54%.

EXAMPLE 4

5′-O-(Phosphonomethyl)thymidine-2′,3′-dideoxy-didehydrothymidine

Step A:

5′-O-(Diethoxyphosphorylmethyl)-2′,3′-dideoxy-didehydrothymidine

50 mmol of sodium hydride (1.5 g of a 80% suspension in oil) are added portionwise to a stirred solution of 22.3 mmol (5 g) of 2′,3′-dideoxy-didehydrothymidine (prepared as described in J. Med. Chem. 32, 461, 1989) in 20 ml of anhydrous dimethylformamide at 20°C under an argon atmosphere. The reaction mixture is stirred at 20°C for 1 hour and 7.4 g (23 mmol) of diethyl p-toluenesulfonyloxymethyl phosphonic acid diethyl ester dissolved in 10 ml of anhydrous dimethylformamide are added to the reaction mixture which is stirred at 20°C for two days. The reaction mixture is evaporated to dryness under reduced pressure. The residue is directly purified by flash chromatography on silica gel (chloroform, ethanol) to give 10 mmol (3.7 g) of the expected product (43%).

Step B:

5′-O-(Phosphonomethyl)thymidine-2′,3′-dideoxy-didehydrothymidine

40 mmol (6.12 g) of trimethylsilylbromide are slowly added to a stirred suspension of 5′-O-(diethoxyphos-

phorylmethyl)-2′,3′-dideoxy-didehydrothymidine (10 mmol, 3.7 g) in 40 ml of anhydrous dimethylformamide at 20°C under argon. The reaction mixture is stirred at 35°C for 5 hours. Dimethylformamide is evapored by distillation under reduced pressure. The residue is dissolved in anhydrous acetonitrile (35 ml) and the final product is obtained on crystallization by addition of water (0.75 ml). The white crystals are collected by filtration and dried at 60°C under vacuum, giving 2.5 mmol (800 mg) of the expected product (25%).

EXAMPLE 5

5′-O-(4-Phosphono-4,4-difluorobutyl)-2′,3′-dideoxyinosine

Step A:

1,1-Difluoro-4-bromobutylphosphonic acid diethyl ester

50 mmol of difluoromethylphosphonic acid diethyl ether dissolved in 50 ml of anhydrous tetrahydrofuran are slowly added to a solution of 50 mmol of lithium diisopropylamine in 5 ml of tetrahydrofuran at -78°C under argon. The reaction mixture is stirred for 45 minutes at -78°C and a cooled (-78°C) solution of 1,3-dibromopropane (100 mmol) in 50 ml of tetrahydrofuran is rapidly (5 minutes) added via a syringe. The reaction mixture is stirred at -78°C for 3 hours, at -20°C for 2 hours, and quenched by addition of 50 ml of saturated aqueous ammonium chloride. Tetrahydrofuran is removed by evaporation under reduced pressure and the residue is extracted three times with 150 ml portions of ethyl acetate. The organic layers are combined, washed with 40 ml of brine, dried over sodium sulfate, filtered and evaporated to give 26 g of a crude oil which is purified by flash chromatography on silica gel giving 24 mmol (7.4 g) of the expected product (48%).

Step B:

5′-O-[4-(Diethoxyphosphoryl)-4,4-difluorobutyl]-2′,3′-dideoxyinosine

70 mmol of sodium hydride (2.1 g of a 80% suspension in oil) are added portionwise to a stirred suspension of 35 mmol of 2′,3′-dideoxyinosine in 35 ml of anhydrous dimethylformamide at 0°C under argon. After 2 hours at -78°C, 24 mmol of 1,1-difluoro-4-bromobutylphosphonic acid diethyl ester dissolved in 10 ml of dimethylformamide are slowly added to the reaction mixture which is stirred at 20°C for 2 days. Dimethylformamide is evaporated under reduced pressure to give 17 g of a residue which is suspended in 25 ml of aqueous saturated ammonium chloride and extracted five times with 60 ml portions of ethyl acetate. The organic layers are dried over sodium sulfate, filtered and evaporated to give 7 g of a residue which is purified by flash chromatography on silica gel (chloroform/ ethanol) to give 3.89 g of the expected product (35% yield).

Step C:

5′-O-(4-Phosphono-4,4-difluorobutyl)-2′,3′-dideoxyinosine

The final product is obtained by the method described in Example 4, Step B.

EXAMPLE 6

5′-O-[2-O-(Phosphonomethyl)-2-hydroxyethyl]-3′-azido-3′-deoxythymidine

Step A:

5′-(Diethoxyphosphoryl-fluoromethylene)-3′-fluoro-2′,3′,5′-trideoxy-5-chlorouridine

A solution of 3′-fluoro-2′,3′-dideoxy-5-chlorouridine (15 mmol, 3.96 g) and dicyclohexylcarbodiimide (9.38 g, 45 mmol) in dimethylsulfoxide (68 ml) was treated with pyridine (1.3 ml, 15 mmol) followed by trifluoroacetic acid (0.6 ml, 3.3 mmol) and the reaction mixture was stirred for 24 hours at room temperature. After removal of the precipitated dicyclohexylurea by filtration, the filtrate was slowly added to a stirred solution of 35 mmol of tetraethyllithiofluoromethylene bisphosphonate dissolved in 50 ml of anhydrous tetrahydrofuran at -78°C under argon. The reaction mixture was stirred at -78°C for 1 hour and at 20°C for 20 hours before it was evaporated to dryness *in vacuo* and directly purified by flash chromatography on silica gel using chloroform and

increasing concentrations of ethanol as eluent to give 6.5 mmol of the expected product (2.7 g, 43%).

Step B:

5′-(Diethoxyphosphoryl-fluoromethyl)-3′-fluoro-2′,3′,5′-trideoxy-5-chlorouridine

To a mixture of the olefin (2.7 g, 6.5 mmol) and potassium azodicarboxylate (6.6 g, 34 mmol) in pyridine (60 ml) was added dropwise with stirring under an argon atmosphere a solution of 2.92 ml (50.8 mmol) of acetic acid in 14 ml of pyridine. The mixture was stirred for 24 hours before it was filtered into 1 L of water, which was extracted 5 times with 50 ml portions of chloroform. Evaporation of the dried chloroform extract gave 2.1 g of partially reduced material. A solution of this material in ethanol (40 ml) containing 400 mg of 5% palladium on barium sulfate catalyst was reduced overnight at atmosphere pressure. The catalyst was removed by filtration and the filtrate evaporated to dryness *in vacuo* to give a yellow oil which was purified by flash chromatography on silica gel (chloroform and increasing concentrations of ethanol as eluents) to give 4.5 mmol of the expected product 5′-(diethoxyphosphoryl-fluoromethyl)-3′-fluoro-2′,3′,5′-trideoxy-5-chlorouridine (1.88 g) 70% yield.

Step C:

5′-(Phosphonofluoromethyl)-3′-fluoro-2′,3′,5′-trideoxy-5-chlorouridine

The final product is obtained by the method described for the preparation of Example 4, Step B.

EXAMPLE 7

5′-O-[2-O-(Phosphonomethyl)-2-hydroxyethyl]-3′-azido-3′-deoxythymidine

Step A:

Synthesis of diethyl(2-trifluoromethanesulfonyloxyethoxy)-methane phosphonate

13.4 ml (80 mmol) of triflic anhydride are slowly added to a solution of pyridine (85 mmol, 6.70 g) in 150 ml of anhydrous dichloromethane at -10°C under argon. The white suspension is stirred at -10°C for 20 minutes and 40 mmol (8.48 g) of 2-hydroxyethoxymethane phosphonic acid diethyl ester dissolved in 40 ml of dichloromethane are slowly added to the reaction mixture at -20°C. After stirring during 2 hours at -20°C and 1 hour at 20°C, the reaction mixture is poured into iced water and extracted 3 times with 80 ml portions of dichloromethane. The organic layers are collected, washed with brine, dried over sodium sulfate, filtered and evaporated to give a crude oil which is directly purified by flash chromatography on silica gel affording 20 mmol (10.32 g) of the expected triflate (75% yield).

Step B:

5′-O-[2-O-(Diethoxyphosphorylmethyl)-2-hydroxyethyl]-3′-azido-3′-deoxythymidine

70 mmol of sodium hydride (2.1 g of a 80% solution in oil) are added portionwise to a stirred suspension of 35 mmol of 3′-deoxy-3′-azidothymidine in 35 ml of anhydrous dimethylformamide at 0°C under argon. After 2 hours at -78°C 30 mmol of the previously described triflate derivative dissolved in 10 ml of dimethylformamide are slowly added to the reaction mixture which is stirred at 20°C for 40 hours. Dimethylformamide is evaporated under reduced pressure to give 16 g of a residue which is suspended in 25 ml of saturated aqueous ammonium chloride and extracted five times with 60 ml portions of ethyl acetate. The organic layers are combined, dried over sodium sulfate, filtered and evaporated to give 9 g of a crude product which is purified by flash chromatography on silica gel (choroform, ethanol) to give 6.7 g of the expected product (42% yield).

Step C:

5′-O-[2-O-(Phosphonomethyl)-2-hydroxyethyl]-3′-azido-3′-deoxythymidine

The final product is obtained by the method described for the preparation of Example 4, Step C.

EXAMPLE 8

5'-(Phosphonodifluoromethyl)-3'-azido-5',3'-dideoxythymidine

Step A:

5'-Trifluoromethanesulfonyl-3'-azido-3'-deoxythymidine

Triflic anhydride (6.7 ml, 40 mmol) is slowly added under an atmosphere of nitrogen to a cooled (-10°C) and stirred solution of pyridine (3.5 ml, 44 mmol) in 120 ml of dichloromethane. The white suspension is stirred for 10 minutes at -10°C and then cooled to -20°C, when a solution of 3'-azido-2',3'-dideoxythymidine (4.58 g, 30 mmol) in anhydrous tetrahydrofuran is slowly added. Stirring is continued for 2 hours at -20°C and 1 hour at room temperature. Crushed ice is added, phases separated, and the aqueous layer extracted with dichloromethane (2 x 50 ml). The combined organic phases are washed with brine, dried (magnesium sulfate) and evaporated (20 Torr, 30°C) to afford an oil. Flash chromatography on silica gel (230-400 mesh, eluent ethyl acetate:petroleum ether 1:1) and evaporation of the product-containing fractions gives 9.6 a (80%) of the title compound as an oil.

Step B:

5'Diethoxyphosphoryldifluoromethyl)-3'-azido-5',3'-dideoxythymidine

A solution of difluoromethylphosphoric acid diethyl ester (9.4 g, 50 mmol) in 50 ml of anhydrous tetrahydrofuran is added slowly under an atmosphere of nitrogen to a cooled (-78°C) and stirred solution of lithiumdiisopropylamide (prepared from 52 mmol butyllithium and 52 mmol diisopropylamine) in anhydrous tetrahydrofuran (50 ml). The reaction mixture is stirred for 45 minutes at -78°C and the precooled (-78°C) solution of the above-prepared triflate (9.6 g, 24 mmol) in 50 ml of anhydrous tetrahydrofuran is rapidly added via a syringe. Stirring is continued for 16 hours at -78°C. A saturated solution of ammonium chloride is added and the mixture allowed to warm up to room temperature. Solvents are evaporated to half of the original volume (20 Torr, 30°C) and the residue is exhaustively extracted with ethyl acetate (3 x 150 ml). The combined organic layers are washed with brine, cried over magnesium sulfate, and evaporated (20 Torr, 30°C) to give an oil which is purified by flash chromatography on silica gel (230 mesh, eluent ethyl acetate). The combined product-containing fractions are evaporated (20 Torr, 30°C, then 0.01 Torr, 20°C) to afford 1.57 g (15%) of the title compound as an oil.

Step C:

5'-(Phosphonodifluoromethyl)-3'-azido-5',3'-dideoxythymidine

At room temperature, trimethylsilylbromide (2.2 g, 14.4 mmol) is added slowly under nitrogen to a stirred suspension of the ester of 5'diethoxyphosphoryldifluoromethyl)-3'-azido-5',3'-dideoxythymicine (1.57 g, 3.6 mmol) in 15 ml of anhydrous dimethylformamide. Stirring is continued at 35°C for 35 hours. The solvent is removed by evaporation under reduced pressure (0.01 Torr, 20-30°C) and the residue dissolved in anhydrous acetonitrile (10 ml). Precipitation of the title compound is caused by addition of water (0.25 ml). Filtration of the mixture and drying of the filter residue (60°C, 0.01 Torr) affords 343 mg (25%) of the final compound.

EXAMPLE 9

5'-O-(4-Phosphono-2-oxobutyl)-3'-fluoro-2',3'-dideoxy-5-chlorouridine

Step A:

5'-O-[4-Diethoxyphosphoryl-2-(tert.butyldimethylsilyloxy)-butyl]-3'-fluoro-2',3'-dideoxy-5-chlorouridine

Sodium hydride (100 mmol, 3.0 g of a 80% suspension in oil) is added portionwise to a stirred solution of 3'-fluoro-2',3'-dideoxy-5-chlorouridine (13.2 g, 50 mmol) in 40 ml of anhydrous dimethylformamide. The reaction mixture is stirred under an atmosphere of nitrogen for 1 hour. A solution of 2-tert-butyldimethylsilyloxy-4-bromobutyl phosphonic acid diethyl ester (20.2 g, 50 mmol) in 20 ml dimethylformamide is added and the resulting mixture stirred at room temperature for two days. Evaporation of the solvent (0.01 Torr, 20-30°C) and

flash chromatography of the oily residue on silica gel (200-400 mesh, eluent chloroform/ ethanol) affords, after evaporation of the pooled product-containing fractions (20 Torr, then 0.1 Torr) 23.4 g (40%) of the desired phosphonate.

Step B:

5′-[4-(Diethoxyphosphoryl)-2-hydroxybutyl]-3′-fluoro-2′,3′-dideoxy-5-chlorouridine

A 1M solution of tetrabutylammonium fluoride in tetrahydrofuran (40 mmol) is added to a stirred solution of 5′-O-[4-diethoxyphosphoryl-2-(tert.butyldimethylsilyloxy)-butyl]-3′-fluoro-2′,3′-dideoxy-5-chlorouridine (23.4 g, 20 mmol) in tetrahydrofuran (100 ml). Stirring of the reaction mixture is continued for 6 hours at room temperature. Then tetrahydrofuran is evaporated and the oily residue applied to flash chromatography on silica gel (230-400 mesh, eluent chloroform/ethanol). Evaporation of the pooled product containing fractions afforded 6.6 g (70%) of the desired alcohol.

Step C:

5′-O-(4-Diethoxyphosphoryl-2-oxobutyl)-3′-fluoro-2′,3′-dideoxy-5-chlorouridine

To a cooled (-55°C) and stirred solution of oxalylchloride (2.54 g, 20 mmol) in anhydrous dichloromethane (20 ml) is added under nitrogen a solution of 3.43 g anhydrous dimethylsulfoxide (44 mmol) in dichloromethane (20 ml) at such a rate that the reaction temperature does not exceed -50°C (5 minutes). Stirring is continued for 15 minutes at -55°C and a solution of 5′-[4-(diethoxyphosphoryl)-2-hydroxybutyl]-3′-fluoro-2′,3′-dideoxy-5-chlorouricine (6.6 g, 14 mmol) in dichloromethane/dimethylsulfoxide (20 ml/l ml) is added over a period of 2 minutes. The reaction mixture is then allowed to warm up to -30°C for 5 minutes and cooled again to -55°C. After 15 minutes stirring at -55°C triethylamine (10 g, 100 mmol) is added at a rate to keep the reaction temperature below -50°C. The mixture is stirred for 20 minutes at -55°C and a saturated solution of aqueous citric acid is added. Having reached room temperature the reaction mixture is diluted with dichloromethane (50 ml), the aqueous layer extracted with dichloromethane (2 x 50 ml). The combined organic phases are washed with brine. Drying over magnesium sulfate and evaporation of the solvents (20 Torr, 30°C) gives an oil which is applied to flash chromatography on silica gel (230-400 mesh, eluent chloroform/ethanol). Evaporation of product-containing fractions affords 1.18 g (60%) of the desired ketone as an oil.

Step D:

5′-O-(4-Phosphono-2-oxobutyl)-3′-fluoro-2′,3′-dideoxy-5-chlorouridine

Saponification of the diethyl phosphonate: 5′-O-(4-di-ethoxyphosphoryl-2-oxobutyl)-3′-fluoro-2′,3′-dideoxy-5-chlorouridine (1.18 g, 8.4 mmol) is performed as described for the synthesis of the phosphonate 5-methyl-1-(2′,3′,4′,5′-tetradeoxy-6′,6′-difluoro-3′-azido-6′-phosphono-ß-D-ribohexofuranosyl)-2,4-(1H,3H)-pyr immidine-dione and affords 1.04 g (30%) of the final compound as a semisolid.

EXAMPLE 10

5′-(4-Phosphoryl-4,4-difluoro-1-butynyl)-5′,3′,2′-trideoxy-quanosine

Step A:

(1,1-Difluoro-4-trimethylsilyl-3-butynyl)phosphonic acid, diethyl ester

A solution of difluoromethyl phosphonic acid diethyl ester (4.7 g, 25 mmol) in anhydrous tetrahydrofuran (25 ml) is added slowly under an atmosphere of nitrogen to a cooled (-78°C) and well stirred solution of lithiumdiisopropylamide (25 mmol) in 50 ml of anhydrous tetrahydrofuran. The reaction mixture is stirred for 45 minutes at -78°C and a precooled (-78°C) solution of 3-trimethylsilyl-1-bromo-2-propyne (4.4 g, 23 mmol) in tetrahydrofuran (25 ml) is added via a syringe. The resulting mixture is then stirred for three hours at -78°C and two hours at -20°C. A saturated solution of aqueous ammonium chloride (20 ml) is added and the mixture evaporated to $\frac{1}{4}$ of its original volume. The mixture is exhaustively extracted with diethyl ether (3 x 50 ml) and the combined organic extracts washed with brine. Drying over magnesium sulfate and evaporation of solvents gives

an oil which is further purified by flash chromatography on silica gel. Evaporation of the product containing fractions affords 2.74 g (40%) of the protected acetylene derivative.

Step B:

(1,1-Difluoro-3-butynyl)phosphonic acid, diethyl ester

A 1M solution of tetrabutylammonium fluoride in tetrahydrofuran (10 mmol) is added to a stirred solution of the above-prepared silyl derivative (1,1-difluoro-4-trimethylsilyl-3-butynylphosphonic acid, diethyl ester), (2.74 g, 9.19 mmol) in tetrahydrofuran (20 ml). Stirring of the reaction mixture is continued for 6 hours at room temperature. Then tetrahydrofuran is evaporated and the oily residue purified by Kugelrohr distillation ($Kp_{0.1}$ : 70-90°C - oven temperature) to afford 1.23 g (60%) of the desired acetylene.

Step C:

5′-[4-(Diethoxyphosphoryl)-4,4-difluoro-1-butynyl]-5′,3′,2′-trideoxyguanosine

Triflic anhydride (2.82 g, 10 mmol) is added under an atmosphere of nitrogen via a syringe to a cooled (0°C) and well stirred solution of 2′,3′-dideoxyguanosine (1.25 g, 5 mmol) and pyridine (0.78 g, 10 mmol) in 50 ml of anhydrous tetrahydrofuran. Stirring is continued for 1 hour at 0°C. Water (5 ml) is added and the solution evaporated to $\frac{1}{4}$ of its original volume. Ethyl acetate (100 ml) is added and phases separated. The organic layer is dried over magnesium sulfate and the solvents evaporated. The resulting triflate (oil) is used as such in the following reaction.

A solution of (1,1-difluoro-3-butynyl)phosphonic acid, diethyl ester (1.23 g, 5.5 mmol) in 10 ml of anhydrous tetrahydrofuran is added via a syringe to a cooled (-78°C) solution of lithiumdiisopropylamide (8.3 mmol) in 10 ml of anhydrous tetrahydrofuran. Stirring at -78°C is continued for 1 hour. Then a solution of the above-prepared triflate in 10 ml of tetrahydrofuran is added slowly via a syringe. Stirring is continued for 30 minutes at -78°C and 30 minutes at -20°C. A saturated solution of ammonium chloride is added and the reaction mixture allowed to warm to room temperature. Solvents are evaporated and the oily residue applied to flash chromatography on silica gel (230-400 mesh, eluent chloroform/ethanol: 13/1). Product-containing fractions are evaporated (20 Torr, 30°C, then 0.01 Torr, 30°C) to afford 1.0 g (40%) of the expected dideoxyguanosine.

Step D:

5′-[4-(Ethoxyphosphinico)-4,4-difluoro-1-butynyl]-5′,3′,2′-trideoxyguanosine

A 1M solution of aqueous sodium hydroxide (4 ml) is added to a stirred solution of the phosphonodiester [5-[(2-amino-1,6-dihydro-6-oxo-9H-purin-9-yl)-2,5-dihydro-2-furanyl]-1,1-difluoro-3-pentynyl]phosphonic acid, diethyl ester (1.0 g, 2.2 mmol) in 5 ml of ethanol. The reaction mixture is stirred for 16 hours at room temperature. The a saturated solution of citric acid is added to achieve a neutral pH. All solvents are evaporated under reduced pressure and the residual solid treated with hot (-50°C) tetrahydrofuran. The solution is then washed with cold (-0°C) brine and dried (magnesium sulfate). Evaporation affords 380 mg (40%) of the final monoester as an oil.

EXAMPLE 11

5′-O-(Hydroxymethylphosphinico)-3′-azido-3′-deoxythymidine

Step A:

t-Butyldiphenylsilyloxymethyl phosphonic acid, diethyl ester

A solution of t-butyldiphenylsilyl chloride (6.6 mmol, 1.72 ml) in 2 ml of anhydrous dimethylformamide is added to a solution of hydroxymethyl phosphonic acid, diethyl ester (6 mmol, 1 g) and imidazole (13.2 mmol, 0.9 g) in 10 ml of dimethylformamide. The reaction mixture is stirred for 12 hours at room temperature. The solvent is then evaporated. The expected compound is obtained by flash chromatography on silica gel (230-400 mesh, eluent ethylacetate/light petroleum: 3/7) in 99% yield as an oil (2.41 g).

Step B:

t-Butyldiphenylsilyloxymethyl, phosphonic acid

A solution of t-butyldiphenylsilyloxymethyl phosphonic acid, diethyl ester (4.92 mmol, 2 g) in bromotrimethylsilane (2 eq., 9.84 mmol, 1.28 ml) is stirred 12 hours under anhydrous conditions. The excess bromotrimethylsilane is removed by evaporation and the residue is stirred withl10 ml of water for 15 minutes. The solution is extracted with ethylacetate, the organic layers are dried over magnesium sulfate, filtered, evaporated to give a crude oil which is purified by flash chromatography on silica gel (methanol/ethylacetate: 2/8) affording an oil that crystallizes by standing (1.64 g, 95% yield).

Step C:

5′-O-(t-Butyldiphenylsilyloxymethylphosphinico)-3′-azido-3′-deoxythymidine

t-Butyldiphenylsilyloxymethyl phosphonic acid (0.75 mmol, 0.242 g) is dissolved in 2 ml of anhydrous pyridine and the solvent is removed *in vacuo* at 35°C. This procedure is repeated three times to provide an anhydrous reaction mixture. The residue is dissolved in 10 ml of anhydrous pyridine, and dicyclohexylcarbodiimide (1.5 mmol, 0.155 g) is added. After 20 hours at room temperature under a nitrogen atmosphere, 30 ml of water are added and the mixture is stirred for 0.5 hour, the dicyclohexylurea is the.n filtered off and the solvents evaporated. The residue is purified by flash chromatography on silica gel (230-400 mesh eluent methanol/chloroform/water: 30/70/3 affording the expected compound as a white solid (0.230 g, 56% yield).

Step D:

5′-O-(Hydroxymethylphosphinico)-3′-azido-3′-deoxythymidine

To a solution of 5′-O-(t-butyldiphenylsilyloxymethyl, phosphinico)-3′-azido-3′-deoxythymidine (0.35 mmol,0.215 g) in 30 ml of anhydrous tetrahydrofuran is added (0.7 mmol, 0.218 g) of tetrabutylammonium fluoride. The reaction mixture is stirred for 0.5 hour at room temperature, the solvent is evaporated and the residue is purified by ion exchange chromatography on DOWEX AG 1X4 ($HCOO^-$ form) affording the expected compound as a white solid (0.70 g, 53% yield).

The compounds of this invention are useful as the active components of medicaments useful for the treatment or prophylaxis of viral infections such as for example hepatitis B, retroviral infections, especially AIDS, respiratory syncitial viruses, and herpes viruses such as herpes simplex I and II, cytomegalovirus and varicella-zoster-virus, as well as antimicrobial and anti-tumoral agents, including tumors in experimental animals.

Examples of retroviral infections which may be treated or prevented in accordance with the invention include human retroviral infections such as Human Immunodeficiency Virus (HIV), HIV-2 and Human T-cell Lymphotropic Virus (HLTV) e.g. HTLV-I or HTLV-IV infections. The compounds according to the invention are especially useful for the treatment or prophylaxis of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive and HIV-positive conditions, Kaposi's sarcoma and thrombocytopenic purpura. The compounds may also be used in the treatment or prevention of psoriasis.

In a further aspect of the present invention there is included a use of a compound according to the invention in the manufacture of a medicament for the treatment or prophylaxis of any of the above-mentioned infections or conditions. "Subject" means any mammal, including a human being and, as described hereafter, fish.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range of 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1,500 mg, preferably 20 to 1,000 mg, and most preferably 50 to 700 mg of active ingredient per unit dosage form.

Ideally, the active ingredient should be administered to achieve peak plasma concentrations of the active compound of from about 1 to about 75μM, preferably about 2 to 50μM, most preferably about 3 to about 30μM. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

The exact regimen for administration of the compounds and compositions disclosed herein will necessarily be dependent upon the needs of the individual subject being treated, the type of treatment and, of course, the judgement of the attending physician.

While it is possible for the active ingredient to be administered alone it is preferable to present it as a pharmaceutical formulation. The formulations of the present invention comprise at least one active ingredient, as defined above, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredients with the carrier which constitutes one or more accessory ingredients. In general, the formulations are prepared by uniformly and intimately bringing into association the active ingredient with liquid-carriers or finely divided solid carriers or both, and then if necessary shaping the product.

Formulations of the present invention suitable for oral administration may be presented is discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste. A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxydants, buffers, bacteriostats and solutes which render the formulation isotonic with the blood of the intended recipient; and aqueous and non-aqueous sterile suspensions which may include suspending agents and thickening agents. The formulations may be presented in unit-dose or multidose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above-recited, or an appropriate fraction thereof, of an active ingredient.

Fish which are in a confined area such as a pool, aquarium or holding tank may also be treated for viral infections such as herpes-like viruses, e.g., channel catfish virus (CCV), herpes-virus salomones, Nerka virus and the like by adding the compound directly to the water of the pool, aquarium, or holding tank or by incorporating the compounds into the feed.

A generally recognized compendium of such methods and ingredients for pharmaceutically acceptable formulations is the latest edition of Remington's Pharmaceutical Sciences, by E.W. Martin (Mack Publishing Co.).

As is true for many large classes of compounds suitable for use as therapeutic agents, certain sub-classes and certain specific members of the generic class will be found to be more effective than others. In this instance the preferred nucleic bases are thymine, adenine, guanine, cytidine and 5-chlorouracil. Preferable $R_1$ moieties are OH and alkyl. Preferred Z-V-W- moieties are $CH_2O$-, CH=CH-, $CH_2OCH_2CH_2O$-, $CF_2C(O)CH_2CH_2$-, and CH-$=CHCH_2CH_2$. Preferably $Y_4$ is H or $N_3$, $Y_3$ is OH, H, F or $N_3$, and $Y_1$ and $Y_2$ are preferably H.

Specific compounds which are preferred are those compounds of the following chart as well as their halomethyl analogs wherein the OH group of $R_1$ is replaced with $CH_{3-x}F_x$.

| $R_1$ | Z-V-W | $Y_4$ | $Y_3$ | $Y_2$ | $Y_1$ | Base |
|---|---|---|---|---|---|---|
| OH | $-CH_2O-$ | $N_3$ | OH | H | H | Thymine |
| OH | -CH=CH- | H | H | H | H | Adenine |
| OH | $CH_2O-CH_2CH_2O-$ | H | $N_3$ | H | H | Guanine |
| OH | $CH_2O-CH_2-CH_2O-$ | $N_3$ | OH | H | H | Thymine |
| OH | -CH=CH- | H | H | H | H | Cytidine |
| OH | $CF_2-\underset{\underset{O}{\Vert}}{C}CH_2CH_2O$ | H | F | H | H | 5-Chlorouracil |
| OH | $CF_2-\underset{\underset{O}{\Vert}}{C}CH_2CH_2O$ | $N_3$ | OH | H | H | Thymine |
| OH | $-CH=CHCH_2CH_2O-$ | H | $N_3$ | H | H | Thymine |

## Claims

1. Compounds of the formula

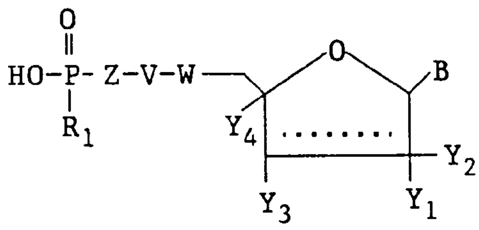

I

their tautomeric forms, their optical and geometric isomers, and mixtures thereof, and the pharmaceutically acceptable salts thereof, wherein the dotted line represents a facultative double bond,
$R_1$ is $C_{1-10}$ alkyl, $CH_{3-x}F_x$ , $C_{1-10}$ alkyl-OH or $OR_2$, x is zero, 1, 2 or 3,
$R_2$ is H or $C_{1-6}$ alkyl,
Z is O, $CH_2O$, $CF_2$, $CCl_2$, CHF, CHCl, CFCl or is deleted,
V is $(CH_2)_m$ or $(CH_2)_m$ Y-$(CH_2)_n$, or is deleted, with m and n being zero, 1, 2, 3 or 4, and Y is -C≡C,

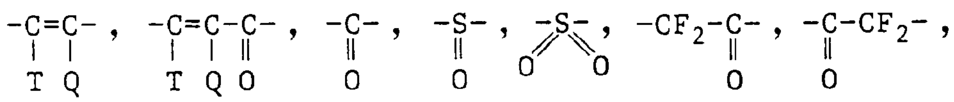

or

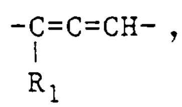

T and Q each are H, F or Cl,
W is O or is deleted,
$Y_1$ is H, Cl, F or OH,
$Y_2$ is H, Cl or F, with the proviso that when the dotted line represents a double bond, then $Y_3$ is F, Cl, or H, $Y_1$ is H, F or Cl and $Y_2$ is deleted,
$Y_3$ is $N_3$, F, Cl, OH or H,
$Y_4$ is H or $N_3$, with the proviso that when $Y_3$ is $N_3$, $Y_4$ is H, with the provisos that
(a) at least one of Z, V and W moieties is other than deleted,
(b) the -Z-V-W- moiety is other than oxygen,
(c) the -Z-V-W- moiety does not contain an oxygen to oxygen bond,
(d) when $Y_3$ is $N_3$, and the -Z-V-W- moiety is $CH_2$ or -$CH_2O$-, then $R_1$ is other than $OR_2$,
(e) when B is a pyrimidine nucleic base and $Y_4$ is H, then the -Z-V-W- moiety is other than $CH_2O$, and
(f) when the -Z-V-W- moiety is a $C_{1-5}$ alkylene and both $Y_3$ and $Y_4$ are other than $N_3$, then $R_1$ is other than $OR_2$,
B is a base selected from the group consisting of

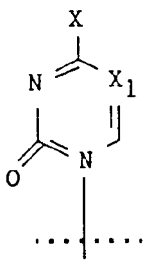
(a)

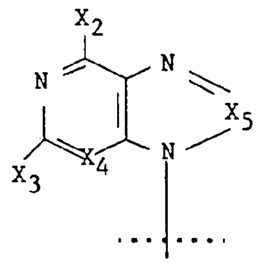
(b)

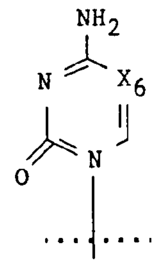
(c)

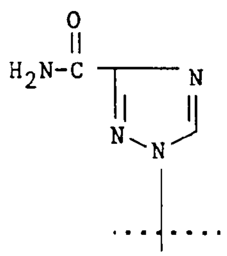
and
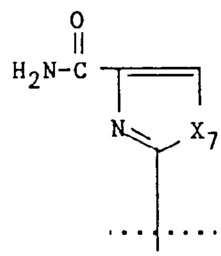

(d) (e)

X is $OR_2$,
$X_1$ is N or C-$Y_7$ with $Y_7$ being H, $CH_3$, $C_2H_5$, I, F, Cl, Br, NHS, SR, -C≡CH, $CH=CH_2$, CH=CHBr, $CH_2CH_2Cl$, -$CH_2CH_2F$, or $CH_2C_6H_4OCH_2C_6H_5$ (meta), and R is H, $CH_3$, $C_2H_5$, $CH_2C≡CH$, -$CH_2CH=CH$ or $CH_2CN$,
$X_2$ is H, OH, Cl, $NH_2$, $SCH_3$ or SH,
$X_3$ is H, $NH_2$, F, Cl, Br or I,
$X_4$ is N or CH,
$X_5$ is N or C-$Y_5$ with $Y_5$ being H, OH, $NH_2$, $NHNH_2$, $CH_3$, Cl, Br, or NHMe,
$X_6$ is C-$Y_6$ with $Y_6$ being H, F, $CH_3$ or CH=CHBr,
$X_7$ is S or Se.

**2.** A compound of Claim 1 wherein B is selected from the group consisting of thymine, adenine, guanine, cytidine and 5-chlorouracil.

**3.** A compound of Claim 1 wherein the -Z-V-W- moiety is $CH_2O$.

**4.** A compound of Claim 1 wherein the -Z-V-W- moiety is CH=CH.

**5.** A compound of Claim 1 wherein the -Z-V-W- moiety is $CH_2OCH_2CH_2O$-.

**6.** A compound of Claim 1 wherein the -Z-V-W- moiety is $CF_2C(O)CH_2CH_2O$-.

**7.** A compound of Claim 1 wherein the -Z-V-W- moiety is $CH=CHCH_2CH_2$.

**8.** A compound of Claim 1 wherein $R_1$ is OH, the -Z-V-W- moiety is $CH_2O$-, $Y_4$ is $N_3$, $Y_3$ is OH, $Y_1$ and $Y_2$ are H, and B is thymine.

**9.** A compound of Claim 1 wherein $R_1$ is OH, the -Z-V-W- moiety is CH=CH, $Y_4$ is $N_3$, $Y_1$, $Y_2$, $Y_3$, $Y_4$ are H, and B is adenine.

**10.** A compound of Claim 1 wherein $R_1$ is OH, the -Z-V-W- moiety is $CH_2OCH_2CH_2O$-, $Y_3$ is $N_3$, $Y_1$, $Y_2$ and $Y_4$ are H, and B is guanine.

**11.** A compound of Claim 1 wherein $R_1$ is OH, the -Z-V-X- moiety is $CH_2OCH_2CH_2O$-, $Y_4$ is $N_3$, $Y_3$ is OH, $Y_1$ and $Y_2$ are H, and B is thymine.

**12.** A compound of Claim 1 wherein $R_1$ is OH, the -Z-V-W- moiety is CH=CH, $Y_1$, $Y_2$, $Y_3$, $Y_4$ are H, and B is cytidine.

**13.** A compound of Claim 1 wherein $R_1$ is OH, the -Z-V-W- moiety is $CF_2C(O)CH_2CH_2O$-, $Y_3$ is F, $Y_1$, $Y_2$ and $Y_4$ are H, and B is 5-chlorouracil.

**14.** A compound of Claim 1 wherein $R_1$ is OH, the -Z-V-W- moiety is $CF_2C(O)CH_2CH_2O$-, $Y_4$ is $N_3$, $Y_3$ is OH, $Y_1$ and $Y_2$ are H, and B is thymine.

**15.** A compound of Claim 1 wherein $R_1$ is OH, the -Z-V-W- moiety is $CH=CHCH_2CH_2O$-, $Y_3$ is $N_3$, $Y_1$, $Y_2$ and $Y_4$ are H, and B is thymine.

**16.** A pharmaceutical composition comprising the compound of claim 1 and a pharmaceutically acceptable carrier.

**17.** The derivatives according to any one of claims 1 to 15 as pharmaceutically active compounds.

**18.** Use of the derivatives according to any one of claims 1 to 15 for the preparation of drugs useful for the treatment or prophylaxis of viral infections.

**19.** Use of the derivatives according to any one of claims 1 to 15 for the preparation of drugs useful as antimicrobial and anti-tumoral agents.

**20.** A process for preparing a compound of the formula

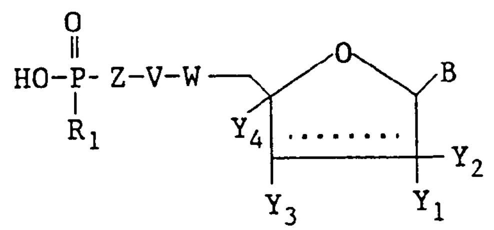

I

their tautomeric forms, their optical and geometric isomers, and mixtures thereof, and the pharmaceutically acceptable salts thereof, wherein the dotted line represents a facultative double bond,
$R_1$ is $C_{1-10}$ alkyl, $CH_{3-x}F_x$ , $C_{1-10}$ alkyl-OH or $OR_2$, x is zero, 1, 2 or 3,
$R_2$ is H or $C_{1-6}$ alkyl,
Z is O, $CH_2O$, $CF_2$, $CCl_2$, CHF, CHCl, CFCl or is deleted,
V is $(CH_2)_m$ or $(CH_2)_m$ Y-$(CH_2)_n$, or is deleted, with m and n being zero, 1, 2, 3 or 4, and Y is -C≡C,

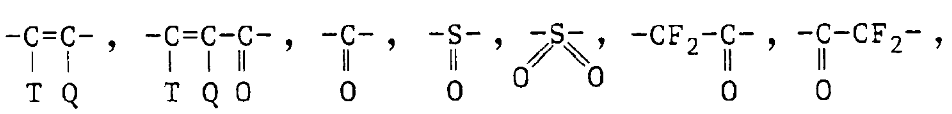

or

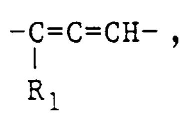

T and Q each are H, F or Cl,
W is O or is deleted,
$Y_1$ is H, Cl, F or OH,

$Y_2$ is H, Cl or F, with the proviso that when the dotted line represents a double bond, then $Y_3$ is F, Cl, or H, $Y_1$ is H, F or Cl and $Y_2$ is deleted,
$Y_3$ is $N_3$, F, Cl, OH or H,
$Y_4$ is H or $N_3$, with the proviso that when $Y_3$ is $N_3$, $Y_4$ is H, with the provisos that
(a) at least one of Z, V and W moieties is other than deleted,
(b) the -Z-V-W- moiety is other than oxygen,
(c) the -Z-V-W- moiety does not contain an oxygen to oxygen bond,
(d) when $Y_3$ is $N_3$, and the -Z-V-W- moiety is $CH_2$ or $-CH_2O-$, then $R_1$ is other than $OR_2$,
(e) when B is a pyrimidine nucleic base and $Y_4$ is H, then the -Z-V-W- moiety is other than $CH_2O$, and
(f) when the -Z-V-W- moiety is a $C_{1-5}$ alkylene and both $Y_3$ and $Y_4$ are other than $N_3$, then $R_1$ is other than $OR_2$,
B is a base selected from the group consisting of

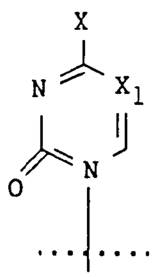
(a)
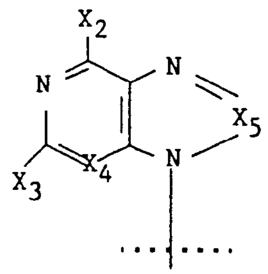
(b)
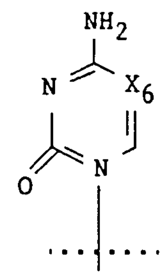
(c)

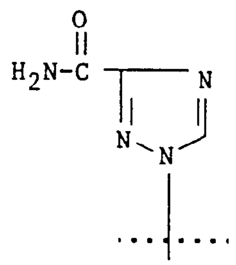
and
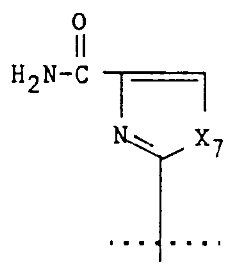
(d) (e)

X is $OR_2$,
$X_1$ is N or C-$Y_7$ with $Y_7$ being H, $CH_3$, $C_2H_5$, I, F, Cl, Br, NHR, SR, -C≡CH, $CH{=}CH_2$, CH=CHBr, $CH_2CH_2Cl$, $-CH_2CH_2F$, or $CH_2C_6H_4OCH_2C_6H_5$ (meta), and R is H, $CH_3$, $C_2H_5$, $CH_2C{\equiv}CH$, $-CH_2CH{=}CH$ or $CH_2CN$,
$X_2$ is H, OH, Cl, $NH_2$, $SCH_3$ or SH,
$X_3$ is H, $NH_2$, F, Cl, Br or I,
$X_4$ is N or CH,
$X_5$ is N or $CY_5$ with $Y_5$ being H, OH, $NH_2$, $NHNH_2$, $CH_3$, Cl, Br, or NHMe,
$X_6$ is $CY_6$ with $Y_6$ being H, F, $CH_3$ or CH=CHBr,
$X_7$ is S or Se,
which comprises
(a) de-esterifying a compound of the formula

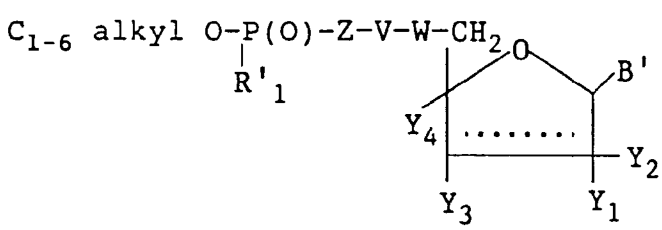

II

the tautomers, optical and geometrical isomers and mixtures thereof, wherein
R′$_1$ is $C_{1-10}$ alkyl, $CH_{3-x}F_x$ , $C_{1-10}$ alkylOalkyl, or OR′$_2$, x is zero, 1, 2 or 3,
R′$_2$ is $OC_{1-6}$ alkyl,
Z, V, W, T, Q, $Y_1$, $Y_2$, $Y_3$ and $Y_4$ are as defined,
B′ is a purine, pyrimidine, triazole or imidazole nucleic base selected from the group consisting of

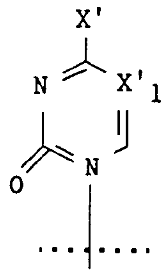
(a)

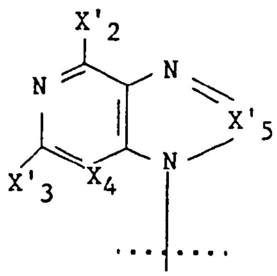
(b)

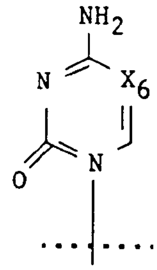
(c)

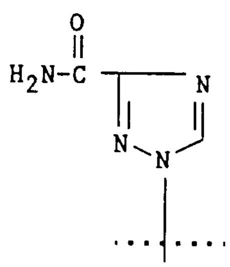
(d)

and

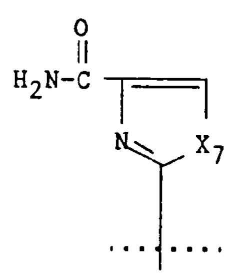
(e)

wherein
X′ is OH, $OR_2$, or a moiety transformable thereto,
X′$_1$ is N or C-$Y_7$ with $Y_7$ being H, $CH_3$, $C_2H_5$, I, F, Cl, Br, NHR, SR, -C≡CH, $CH{=}CH_2$, CH=CHBr, $CH_2CH_2Cl$, $-CH_2CH_2F$, or $CH_2C_6H_4OCH_2C_6H_5$ (meta), and R is H, $CH_3$, $C_2H_5$, $CH_2C{\equiv}CH$, $-CH_2CH{=}CH$ or $CH_2CN$,, or a moiety transformable thereto,
X′$_2$ is H, OH, Cl, $NH_2$, $SCH_3$ or SH,, or a moiety transformable thereto,
X′$_3$ is H, $NH_2$, F, Cl, Br or I, or a moiety transformable thereto,
X′$_5$ is N or C-$Y_5$ with $Y_5$ being H, OH, $NH_2$, $NHNH_2$, $CH_3$, Cl, Br, or NHMe, or a moiety transformable thereto,
$X_4$, $X_6$ and $X_7$ being as previously defined,
by reaction with TMSBr or TMSI, and when necessary chemically transforming a transformable moiety of X′, X′$_1$, X′$_2$, X′$_3$, or X′$_5$ to the desired X, $X_1$, $X_2$, $X_3$, or $X_5$ moiety, respectively, or
(b) chemically transforming a compound of Formula II, to the desired X, $X_1$, $X_2$, $X_3$, or $X_5$ moiety, and optionally de-esterifying the phosphonate moiety by reaction with TMSBr or TMSI, followed by optionally forming the desired pharmaceutically acceptable salts thereof.